# EUROPEAN PATENT APPLICATION

(11) **EP 1 985 713 A1**
(43) Date of publication of application: **29.10.2008**
(21) Application number: 07105731.9
(22) Date of filing: 05.04.2007
(51) Int. Cl.: C12Q 1/68

(54) **Methods and tools for detecting the presence of colorectal adenocarcinoma cells**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: van Wermeskerken, Stephanie Christine

(57) **Abstract**

The present invention discloses methods and tools for reliably detecting the presence of adenocarcinoma cells in a patient, at the genetic level. The present invention fine-maps the regions of chromosomal aberrations linked to progression of adenomas into adenocarcinoma cells and provides methods and tools for detection based thereon.

## Description

### FIELD OF THE INVENTION

The present invention relates to methods for tumour diagnosis, more particularly for detecting the presence of colorectal adenocarcinomas. The present invention further provides primers and probes for performing these methods.

### BACKGROUND OF THE INVENTION

Cancer of the colorectal part of the gastrointestinal tract is a frequently occurring disorder. In a first stage a benign tumour (adenoma) occurs which can turn in to a malignant cancer (adenocarcinoma). Not all adenomas progress to carcinomas. In fact this progression into a carcinoma happens in only a small subset of tumours. Initiation of genomic instability is a crucial step and occurs in two ways in colorectal cancer (Lengauer et al. (1998) Nature, 396, 643-649). DNA mismatch repair deficiency leading to microsatellite instability (abbreviated as MSI or MIN), has been most extensively studied (di Pietro et al. (2005) Gastroenterology, 129, 1047-1059), but explains only about 15% of adenoma to carcinoma progression. In the other 85% of cases where colorectal adenomas progress to carcinomas, genomic instability occurs at the chromosomal level (CIN) giving rise to aneuploidy. While for a long time these chromosomal aberrations have been regarded as random noise, secondary to cancer development, it has now been well established that these DNA copy number changes occur in specific patterns and are associated with different clinical behaviour (Hermsen et al. (2002). Gastroenterology 123, 1109-1119). Chromosomal aberrations frequently reported in colorectal cancers are 7pq, 8q, 13q, 20q gains and 4pq, 5q, 8p, 15q, 17p, 18q losses. It was shown that 8q, 13q and 20q gains and 8p, 15q, 17p and 18q losses, are associated with progression of colorectal adenomas to carcinomas (Hermsen et al. (2002) cited above). Gain of chromosome arm 20q is the most frequent gain observed in colorectal cancer, being altered in more than 65% of the cases (Meijer et al. (1998) J. Clin. Pathol. 51, 901-909). Gains on 20q, in particular the region 20q12-q13, are also commonly described in other types of solid tumours and have been associated with poor outcome in both gastric and colorectal cancers. Methods to detect these chromosomal aberrations are generally based on Comparative Genomic Hybridisation (CGH). Rooney et al. (2004 J. Pathol. 204, 282-288), describes the use of a qualitative PCR to detect gene duplication of the gene ZNF217 in colon cancer.

It is of utmost clinical importance to identify the above described progression of adenomas into adenocarcinomas in as early a stage as possible, to allow early stage treatment of carcinomas while avoiding unnecessary surgical intervention. Ideally, the adenocarcinomas can be identified at a stage where the presence of malignant cells is not yet detectable by in situ optical analysis or microscopic analysis of biopsy or resection material.

### SUMMARY OF THE INVENTION

The present invention relates to methods and tools for cancer diagnosis, more specifically for the identification of the presence of adenocarcinomas in the colon and/or the rectum. More particularly, the present invention provides methods and tools which allow the differential detection of carcinomas with increased sensitivity and reliability.

Adenomas may or may not develop into an adenocarcinoma. However, it is an advantage of the present invention to detect malignant colorectal adenocarcinoma cells e.g. within an adenoma, at a very early stage based on the differential genetic makeup of these cells. This screening can be performed using very sensitive techniques such that the presence of only a small number of carcinoma cells can be detected. This allows identification of the presence of carcinoma cells, prior to a stage wherein they can be detected by echography, radiography or MRI (Magnetic Resonance Imaging) and allows the detection of adenocarcinoma cells in material which can be obtained from the colon without the need for a biopsy or resection (e.g. stool).

The present invention is based on the finding that the progression of a colorectal adenoma into an adenocarcinoma is linked to the appearance of at least one region of chromosomal gain or loss within a chromosome of an adenoma cell. Accordingly the present invention aims to accurately detect the presence of carcinoma cells by detecting these gains or losses by direct or indirect analysis methods.

In one aspect of the invention methods are provided for the detection of the presence of colorectal adenocarcinoma cells in a patient based on detecting the presence of genetic material comprising a chromosomal gain or loss which is correlated with the progression from a colorectal adenoma into an adenocarcinoma.

More particularly, in vitro methods are provided for detecting the presence of colorectal adenocarcinoma cells in a patient, which methods comprise the steps of detecting the presence of gain or loss of one or more smallest region of overlap (SRO) in the genomic DNA of a test sample of the patient, wherein the presence of genetic material comprising a gain or loss of one or more of SRO is indicative of the presence of colorectal adenocarcinoma cells in the patient. More particularly, in the methods of the present invention, the one or more SRO is selected from:
- the group consisting of SRO_8p_1, SRO_8p_2, SRO_8p_3 and SRO_8p_4 on chromosome 8, and/or
- the group consisting of SRO_8q_1, SRO_8q_2, SRO_8q_3, SRO_8q_4, SRO_8q_5 and SRO_8q_6 on chromosome 8, and/or
- the group consisting of SRO_13q_1, SRO_13q_2, SRO_13q_3, SRO_13q_4, SRO_13q_5 and SRO_13q_6 on chromosome 13 q, and/or
- the group consisting of SRO_15q_1, SRO_ 15q_2 and SRO_15q_3 on chromosome 15, and/or
- the group consisting of SRO_17p_1 and SRO_17p_2 on chromosome 17, and/or
- the group consisting of SRO_18q_1 and SRO_18q_2 on chromosome 18, and/or
- the group consisting of SRO_20q_1, SRO_20q_2 and SRO_20q_3 on chromosome 20.

In particular embodiments, the methods of the present invention encompass the detection of gain or loss of at least one SRO of each of the regions of chromosomal gain or loss linked to colorectal adenocarcinomas, more particularly chromosomal gain at 8q, 13q, and 20q, and chromosomal loss at 8p, 15q, 17p and 18q. In further embodiments, the methods of the present invention encompass the detection of gain or loss for all of said SROs of at least one chromosomal region. In further embodiments, the methods of the present invention encompass the detection of gain or loss for all of said SROs of each of the listed chromosomal regions.

In particular embodiments, the methods of the present invention encompass the detection of chromosomal gain or loss using Comparative Genomic Hybridisation (CGH), in particular array CGH.

In further particular embodiments the methods of the present invention encompass the detection of chromosomal gain or loss using techniques such as fluorescent in situ hybridisation, Southern blots or loss of heterozygosity analysis.

In further particular embodiments, the methods of the present invention encompass the detection of chromosomal gain or loss by a PCR reaction with SRO-specific primer pairs. In particular embodiments the SRO-specific primer pair used is located close to the boundaries of the SRO, and allows the detection of the loss or gain of a SRO as disclosed in the present invention. Both quantitative and qualitative PCR methods are envisaged. More particularly, the SRO-specific primer pair for the detection of an SRO of a region of chromosomal loss comprises a forward primer located 5' and a reverse primer located 3' from said SRO. In particular embodiments the SRO-specific primer pair for the detection of an SRO within a region of chromosomal gain comprises a forward primer within the 3' region of said SRO and a reverse primer within the 5' region of the SRO.

In further particular embodiments, the methods of the present invention encompass the detection of chromosomal gain or loss by a quantitative PCR detection of a sequence located within the one or more SROs.

In further particular embodiments the methods of the present invention encompass the detection of chromosomal gain or loss using MPLA (multiplex ligation dependent probe amplification).

In particular embodiments, the methods of the present invention for detecting the presence of colorectal adenocarcinoma cells in a patient are carried out on a test sample of the patient which is a sample comprising material from a colorectal tumour biopsy or resection. Alternatively, the test sample comprises material from a stool sample. Further alternatives envisaged are methods carried out on test samples which comprise material from a tissue or fluid selected from the group consisting of blood, urine, saliva, and colon fluid.

The methods of the invention are particularly suited for diagnosing the progression of a colorectal adenoma into an adenocarcinoma.

Another aspect of the invention provides kits for the detection of chromosomal deletions or duplications, which comprise, for one or more SROs of each of the chromosomal regions depicted in Table 1, one or more oligonucleotides hybridising specifically under stringent conditions with a sequence within said SRO or with a sequence within the genomic sequence up to about 1 Mb located 3' or 5' from the boundaries of said SRO. In particular embodiments, the one or more oligonucleotides are a pair of SRO-specific PCR primers. In further embodiments, the one or more oligonucleotides are SRO-specific probes. In further specific embodiments the SRO-specific primers detect one or more of the marker genes of Table 2.

Accordingly, a further aspect of the present invention relates to the use of the kits provided herein for the in vitro determination of the presence of colorectal adenoma cells in a sample of a patient. More particularly the kits are used in a method for determining a chromosomal aberration in cells of a colorectal lesion. Where the presence of adenoma cells has been identified, the methods and kits of the present invention can be used for the in vitro determination of the progression of a colorectal adenoma into an adenocarcinoma.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference Figure quoted below refer to the attached drawings.

### BRIEF DESCRIPTION OF THE DRAWING

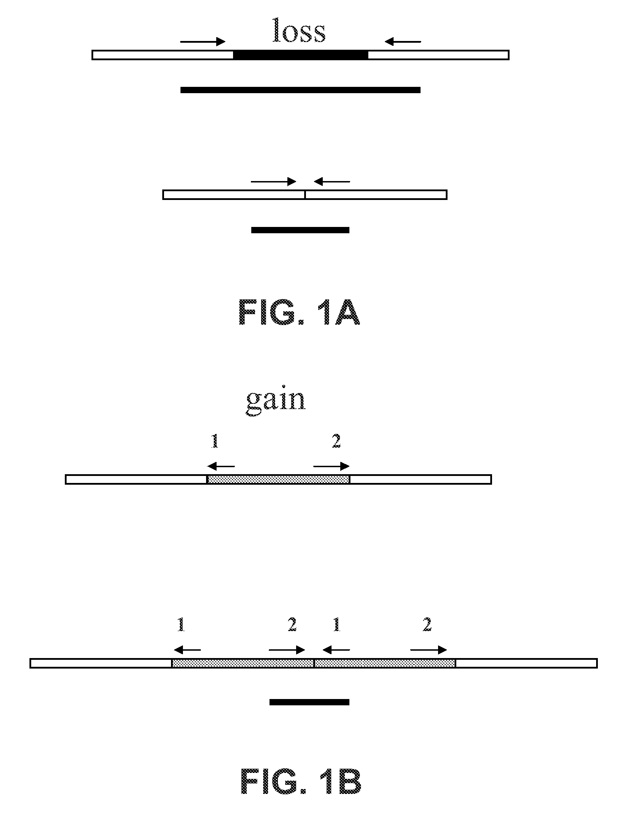

Fig. 1 schematically illustrates the principle of detecting chromosomal loss (A) or gain (B) in a polynucleotide sequence using a qualitative PCR reaction. The Figure shows a part of genomic DNA before and after the chromosomal aberration. Arrows represent PCR primers. The length of the PCR fragments (if generated) is shown below the genomic DNA.

### DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### Definitions

"Tumour" or "neoplasm" as used herein refers to an abnormal tissue that grows by cellular proliferation more rapidly than normally, and continues to grow after the stimuli that initiated the new growth cease. The term "lesion", generally referring to an abnormality involving any tissue or organ due to any disease or any injury, is also used herein to refer to a neoplasm. Tumours, neoplasm or lesions can be either benign or malignant.

"Cancer" is a general term referring to any type of malignant neoplasm.

"Adenoma", as used herein, relates to a benign epithelial neoplasm. Adenomas are usually well circumscribed, they can be flat or polypoid and the neoplastic cells do not infiltrate or invade adjacent tissue.

"Adenocarcinoma", as used herein, relates to a malignant neoplasm of epithelial cells. Most commonly carcinoma's form a glandular or glandlike pattern. Synonyms are "glandular cancers" and "glandular carcinomas". Malignant cells are often characterized by progressive and uncontrolled growth. They can spread locally or though the blood stream and lymphatic system to other parts of the body. Colorectal adenomas are common in the elderly population, but only a small proportion of these pre-malignant tumours (estimated approximately 5%) progresses to malignant tumours (i.e. colorectal adenocarcinoma).

"Progressed adenomas" are adenomas that harbour already a focus of a cancer and also called "malignant polyps".

"Colorectal" relates to of the colon and/or the rectum, i.e. the complete large intestine.

"Chromosomal aberration", as used herein refers to a chromosomal loss or gain, i.e. a region in the chromosome which has been deleted or duplicated.

"Marker gene", as used herein refers to a gene of which expression differs (decreased or increased) between adenoma and adenocarcinoma cells.

"Expression profile" refers to the expression level of a number of a marker genes in a cell or a sample.

The next best thing to colon cancer prevention is early diagnosis. There are five stages of colon cancer (0-4). This staging system reflects the infiltration stage of the cancer. In general, the earlier the stage, the easier the cancer is to treat.

The present invention provides diagnostic methods and tools for distinguishing between malignant and pre-malignant colorectal lesions and for diagnosing the presence of colorectal cancer in a subject. For example, the diagnostic methods of the present invention can reliably detect very early-stage colorectal cancers.

While colonoscopy and sigmoidoscopy are the most commonly used examinations for early detection of colorectal cancer, they are not only quite uncomfortable for the patient, but they only allow the detection of visible lesions. Moreover, their sensitivity is determined by the experience of the doctor performing the test. The taking of resections or biopsies during colonoscopy is usually performed when polyps are identified to confirm the nature of the polyp by histological analysis. More recently, DNA microarray-based tumor gene expression profiles have been used for cancer diagnosis. However, studies have been limited to a few cancer types and have spanned multiple technology platforms complicating comparison among different datasets. Expression analysis performed at the RNA or protein level has a number of drawbacks, the first being linked to the origin of the samples, i.e. the need for an invasive colonoscopy. RNA or protein samples are moreover easily contaminated by the content of the colon or rectum which complicates the subsequent analysis or leads to degradation of the material to be assayed. Also, especially for early diagnosis where a sample may contain only a small number of adenocarcinoma cells compared to the cell mass of the surrounding adenoma tissue, the contribution of the aberrant expression of genes by such a small number of adenocarcinoma cells may not be noticed. Thus the sensitivity of expression analysis is limited.

The present invention is based on the observation that as over 85% of colorectal carcinomas are linked to the occurrence of chromosomal aberrations, it should be possible to detect these carcinomas at the genetic level, avoiding the disadvantages of protein or RNA expression analysis. In one aspect, the present invention relates to in vitro methods wherein genomic DNA is used for the detection of colorectal adenocarcinoma cells in a patient by analysing directly the presence or absence of chromosomal aberrations in the genetic material of the sample. Colorectal adenocarcinoma cells have been found to show chromosomal gain at 8q, 13q, and 20q, while chromosomal loss is encountered at 8p 15q, 17p or 18q. The presence of genetic material comprising one or more of these chromosomal aberrations in a sample of a patient, is indicative of the presence of colorectal adenocarcinoma cells in that patient. As the occurrence of these chromosomal aberrations are characteristic of adenocarcinoma cells vs. non-malignant adenoma cells, the methods of the invention accordingly allow the detection of the progression of a benign colorectal adenoma into a malignant carcinoma.

The patient sample used in the methods of the present invention is a sample comprising genomic DNA, more particularly potentially comprising genomic DNA originating from carcinoma cells present in the patient. In one embodiment, the sample comprises colorectal tissue of the patient, more particularly the sample is or comprises genetic material from a biopsy or resection of a colorectal lesion. However, in view of the sensitivity of the methods of the present invention, samples comprising a very small amount of genetic material can also be used. In particular embodiments, the genomic analysis of the methods of the invention is performed on material from the body that contains intact cells or lysed cells from a colorectal lesion, such as a stool sample. Adenocarcinoma cells can invade other tissues. Therefore, the methods of the present invention also envisage the analysis of test samples other than from colorectal biopsies or resections and stool samples. The analysis of samples such as blood, urine, saliva which can contain adenocarcinoma cells or at least parts of the genomic DNA of these cells is also envisaged.

In some embodiments, the methods of the present invention comprise comparing the genomic analysis of a sample obtained from a patient to a control. Suitable controls in the context of the present invention include genomic DNA not comprising genetic material from adenocarcinoma cells. Typically, a suitable control is genetic material from the same patient from a non-affected tissue or from adenoma tissue not comprising adenocarcinoma cells. Control material can also be genetic material originating from another patient or from a pool of control patients (not comprising genetic material from adenocarcinoma cells).

The presence of DNA material comprising chromosomal gain at 8q, 13q, and 20q, and/or chromosomal loss at 8p, 15q, 17p or 18q, genomic DNA can be analysed by any number of techniques of genetic analysis. Loss of heterozygosity (LOH) can be performed using polymorphic markers. Alternatively, hybridisation methods such as CGH (Comparative Genomic Hybridisation) are used.

According to one embodiment, the detection of chromosomal deletions and insertions in the methods of the present invention is performed using CGH. Herein genomic DNA of a test sample is hybridised with an array of genomic clones representing the human genome. CGH is an established method, an example of this procedure is explained in detail in the Examples section of the present application. CGH is based on the hybridisation of sample DNA with DNA on matrix. The presence of genomic aberrations is detected based on a difference in the hybridisation pattern compared to control DNA. In order to have a reliable result, non-specific hybridisation is to be avoided. This is performed e.g. by removing non-specifically bound DNA using elevated temperatures, high salt concentrations and chaotropic agents such as formamide. The values for each of these parameters depend on the degree of sequence similarity and length of the hybridising partners. Suitable values are found in instructions of the manufacturers of CGH arrays and in reference books such as Sambrook et al. (2000) in Molecular Cloning, A Laboratory Manual, third edition, Cold Spring Harbor Press. Examples are solutions containing about 50% formamide, 2x SSC, pH 7 or 0.1 M sodium phosphate, 0.1% Nonidet P40, pH 8 with SSC concentrations ranging from 0.2 to 0.01 x SSC.

By fine-mapping the chromosomal regions that are deleted or duplicated upon transition from adenoma into carcinoma, minimal regions have been identified for the regions of chromosomal loss and gain previously linked to colorectal adenocarcinoma.

Accordingly, particular embodiments of the invention relate to methods for detecting the presence of colorectal adenocarcinoma cells in a sample of a patient which comprise detecting the presence of gain or loss of at least one smallest region of overlap (SRO). Smallest regions of overlap (SRO), have been identified for each of the regions of chromosomal gain, more particularly at 8q, 13q, and 20q, and each of the regions of chromosomal loss, more particularly at 8p, 15q, 17p or 18q, associated with colorectal adenocarcinomas. Indeed, some variation exists in the exact size and location of the chromosomal aberrations that were found to be associated with colorectal adenocarcinomas. SROs correspond to those regions which are always gained or lost when a chromosomal gain or loss occurs at the respective chromosomal region, and thus are representative for the chromosomal loss or gain to which they correspond. The SROs identified for each of the regions of chromosomal loss or gain are provided in Table 1.

**Table 1: Smallest regions of overlap of chromosomal aberrations in colorectal adenocarcinoma cells.**

| Chromosomal Region | Designation SRO | Position (#) |
|---|---|---|
| 8p | SRO_8p_1 | 2-3Mb |
| | SRO_8p_2 | 4-19Mb |
| | SRO_8p_3 | 21-23Mb |
| | SRO_8p_4 | 32-35Mb |
| | | |
| 8q | SRO_8q_1 | 67-69Mb |
| | SRO_8q_2 | 104-105Mb |
| | SRO_8q_3 | 117-118Mb |
| | SRO_8q_4 | 122-126Mb |
| | SRO_8q_5 | 130-131Mb |
| | SRO_8q_6 | 139-147Mb |
| | | |
| 13q | SRO_13q_1 | 24-25Mb |
| | SRO_13q_2 | 30-31Mb |
| | SRO_13q_3 | 45-46 Mb |
| | SRO_13q_4 | 61-62Mb |
| | SRO_13q_5 | 70-80Mb |
| | SRO_13q_6 | 107-114Mb |
| | | |
| 15q | SRO_15q_1 | 12-21Mb |
| | SRO_15q_2 | 50-54Mb |
| | SRO_15q_3 | 72-82Mb |
| | | |
| 17p | SRO_17p_1 | 0-2Mb |
| | SRO_17p_2 | 12-15Mb |
| | | |
| 18q | SRO_18q_1 | 24-26Mb |
| | SRO_18q_2 | 74-77Mb |
| | | |
| 20q | SRO_20q_1 | 32-36Mb |
| | SRO_20q_2 | 56-59Mb |
| | SRO_20q_3 | 61-64Mb |

| | | |
|---|---|---|
| #: localisation of SRO regions on the respective chromosomes, in accordance with the UCSC July 2003 freeze of the Human Golden Path. | | |

Thus, particular embodiments of the methods of detection of the present invention comprise the detection of one or more of the SROs of Table 1, for one or more of the regions of chromosomal loss/gain which have been associated with colorectal adenocarcinomas.

The narrowing of regions of chromosomal aberrations into SROs allows for the detection of well-defined regions of DNA. This SRO-specific detection can be performed, for instance, by (quantitative or qualitative) PCR. The specificity and sensitivity of PCR makes it possible to detect the SROs in a sample containing only a minute amount of cells or DNA or containing only minute amounts of genomic material from the colorectal lesion. For instance, a stool sample may comprises only minute amounts of cellular material released from a colorectal lesion such as an adenomatous polyp.

According to a particular embodiment, the methods of the present invention comprise detecting the one or more SROs described by MLPA. In this technique two probes are used which hybridise adjacent to each other on sample DNA. Hereafter the probes are ligated and the ligated probes, instead of the sample is amplified by PCR. In particular embodiments, the probes are selected such that target sequence of the adjacent probes is a sequence within the SRO. The amount of amplified product reflects the relative copy number of the target sequence. Alternatively probes can be selected such that the size or the presence/absence of the amplicon is indicative of chromosomal aberration. MLPA allows the use of different probe pairs, hybridizing to different parts of a chromosome (each generating an amplicon of a specific length) at the same time. Accordingly, different SROs can be detected simultaneously (Schouten et al. 2002, Nucl. Acids Res. 30(12), e57).

Alternatively, the identification of well-defined genomic regions of interest allows a further refinement of the CGH technique whereby only probes directed to the specific region of interest are used.

Accordingly, in one embodiment, the presence of colorectal adenoma cells in a sample is determined by determining chromosomal loss of one or more or all of the regions on 8p designated as SRO_8p_1, SRO_8p_2, SRO_8p_3, and SRO_8p_4.

Additionally or alternatively, the presence of colorectal adenoma cells in a sample is determined by determining chromosomal gain of one or more or all of the regions on 8q designated as SRO_8q_1, SRO_8q_2, SRO_8q_3, SRO_8q_4, SRO_8q_5 and SRO_8q_6.

Additionally or alternatively, the presence of colorectal adenoma cells in a sample is determined by determining chromosomal gain of one or more, or all of the regions on 13q designated as SRO_13q_1 SRO_13q_2, SRO_13q_3 SRO_13q_4, SRO_13q_5 and SRO_13q_6.

Additionally or alternatively, the presence of colorectal adenoma cells in a sample is determined by determining chromosomal loss of one or more, or all of the regions on 15q designated as SRO_15q_1, SRO_15q_2 and SRO_15q_3.

Additionally or alternatively, the presence of colorectal adenoma cells in a sample is determined by determining chromosomal loss of one or both the regions on 17p designated as SRO_17p_1 and SRO_17p_2.

Additionally or alternatively, the presence of colorectal adenoma cells in a sample is determined by determining chromosomal loss of one or both of the regions on 18q designated as SRO_18q_1 and SRO_18q_2.

Additionally or alternatively, the presence of colorectal adenoma cells in a sample is determined by determining chromosomal gain of one both or all three of the regions on 20q designated as SRO_20q_1, SRO_20q_2 and SRO_20q_3.

Most particularly, the present invention provides methods whereby the presence of colorectal carcinoma cells in a patient can be reliably detected, based on the detecting whether any of the regions of chromosomal loss or gain linked with the occurrence of colorectal cancer can be detected in genomic material of the patient. Thus, a particular embodiment of the present invention refers to methods for detecting adenocarcinoma cells, which comprise determining chromosomal gain or loss of at least one SRO of each of the chromosomal aberrations 8p loss, 8q gain, 13q gain, 15q loss, 17p loss, 18q loss, and 20q gain mentioned in Table 1. In a very particular embodiment, this detection is performed by determining chromosomal gain or loss of all SRO's of each of the chromosomal aberrations 8p loss, 8q gain, 13q gain, 15q loss, 17p loss, 18q loss, and 20q gain mentioned in Table 1. The methods of the present invention allow the reliable detection of the progression of colorectal adenoma cells in a patient into adenocarcinoma cells.

According to one embodiment, the methods of the present invention comprise detecting the one or more SRO described above by SRO-specific primer pairs in PCR amplification techniques. SRO-specific primer pairs are primer pairs which, when used to amplify genomic material, will generate a different amplicon depending on whether or not there is a chromosomal aberration (gain/loss) involving the SRO. Where SRO-specific primer pairs are used, one or more chromosomal gains and/or losses can be identified without the use of quantitative PCR methods.

Accordingly, in a further aspect, the present invention provides primer pairs which detect the duplication or loss of one or more SROs of the regions of chromosomal gain and loss associated with colorectal adenocarcinoma. More particularly, the present invention provides SRO-specific primer pairs for the SROs depicted in Table 1. Additionally the present inventions provides sets of primer pairs suitable for the detection of a combination of SROs according to the present invention, more particularly for the detection of one SRO of each region of chromosomal loss or gain associated with colorectal adenocarcinomas. Further embodiments relate to sets of primer pairs comprising primers for the detection of each of the SROs of the present invention.

Deletions can be qualitatively detected, e.g., by a forward primer located 5' and a reverse primer located 3' from an SRO which of a region of chromosomal loss. When a deletion (or chromosomal loss) of that SRO occurs, a part of the genomic DNA between the primers is absent and results in the generation of a PCR product which is considerably smaller than in wild-type (no chromosomal loss). The PCR fragments amplified from regions with a deletion are smaller than the fragments of an intact chromosome. Such smaller fragments will be amplified preferentially, allowing a very sensitive detection. Additionally or alternatively, the elongation time in a PCR reaction can be shortened to discourage the amplification of longer PCR products.

The occurrence of a duplication, or a chromosomal gain is detected, e.g., by a forward primer in the 3' region and a reverse primer in the 5' region of a SRO as designated in Table 1. Since these primers "point away" from each other, there will be no PCR product at all on a chromosome without duplication. The concept of detecting deletions or duplications is illustrated in Figure 1.

Stringency conditions for use with PCR primers are determined by calculation the length, GC composition and degree of sequence identity between primer and template. Based upon the predicted melting temperature of a primer, the conditions of a PCR amplification are adapted. The stringency parameters in a PCR reaction are largely determined by the choice of the annealing temperature in a PCR cyclus. Different software programs are available to select in a given DNA sequence a pair of PCR primers with desired melting temperature, which are specific and which do not hybridise with each other, or form hairpins. Optionally, the specificity of a PCR reaction in increased by performing so-called nested PCR. Kits for amplification of genomic DNA are available from, for example, Roche or Stratagene.

According to another embodiment, the methods of the present invention comprise detecting the one or more SRO described above by quantitative PCR. Using primers annealing to a sequence located within the SRO of interest, the quantitative expression of this sequence in a sample can be compared to a control (same region in a control sample or other region). Similarly, using MLPA, primer pairs can be used which target a sequence within a region of chromosomal loss or gain (MLPA), resulting in the generation of a relative amount of amplicon which reflects the relative copy number of the target sequence.

A specific target sequence located within the SROs identified in the context of the present invention, more particularly, the SROs identified in Table 1, can be easily determined by the skilled person. While in essence any part of genomic DNA is suitable as a target for amplification, in particular embodiments, a part of a gene, more particularly at least a part of at least one exon is used as target for amplification.

Accordingly, the particular embodiments of the methods of the present invention relate to the detection of the presence of colorectal adenocarcinoma cells in a sample, which methods comprise quantitatively detecting the occurrence of a DNA sequence located within an SRO associated with colorectal adenocarcinoma, more particularly a sequence located within an SRO of Table 1.

According to another embodiment, the methods of the present invention comprise detecting the one or more SRO described above by a genomic screen using probes specific for sequences within an SRO (SRO-specific probes). Similar to the technique of CGH, the hybridization of genomic material comprising a chromosomal aberration with a set of probes specific for a sequence within a SRO will give a different signal compared to control genomic material. The genes located within the SROs identified in the context of the present invention, more particularly, the SROs identified in Table 1 can be easily determined by the skilled person.

Accordingly, in a further aspect, the present invention provides probes which allow the detection, within a sample comprising genomic DNA of the presence of genomic material comprising a duplication or loss of one or more SROs of the regions of chromosomal gain and loss associated with colorectal adenocarcinoma. More particularly, the present invention provides SRO-specific probes for the SROs depicted in Table 1. Additionally the present inventions provides kits comprising probes suitable for the detection of a combination of SROs according to the present invention, more particularly for the detection of one SRO of each region of chromosomal loss or gain associated with colorectal adenocarcinomas, i.e. the regions of chromosomal gain at 8q, 13q, and 20q, and the regions of chromosomal loss at 8p, 15q, 17p or 18q. Further embodiments relate to kits comprising probes for the detection of each of the SROs of the present invention.

Thus, generally, the present invention provides kits comprising one or more oligonucleotides hybridising specifically under stringent conditions with a sequence within a SRO or with a sequence within the genomic sequence up to about 1 Mb located 3' or 5' from the boundaries of said SRO, which allow specific detection of the SRO.

A further aspect of the present invention provides marker genes for the SROs identified for the chromosomal gains and losses associated with colorectal adenocarcinomas. The marker genes of the present invention are genes which are located within the SRO's of the regions of chromosomal loss and gain associated with colorectal carcinomas. More particularly, the marker genes of the invention are genes which are located within the SROs disclosed in Table 1, and which have been shown to have a differential expression between adenoma cells and adenocarcinoma cells. A list of the marker genes of the present invention is provided in Table 2, below. These genes are of particular interest as they allow screening of genomic and expression analysis in parallel.

Accordingly, a further embodiment of the present invention relates to methods for the detection of colorectal adenocarcinoma cells in a patient, which comprise detecting the loss or gain of the genomic DNA of one of one or more of the genes depicted in Table 2. Accession numbers in Table 2 refer to mRNA entries in Genbank. The identifier of the gene is indicated in brackets behind the description. Entry of either gene name or cDNA leads to genomic sequences with indication of the different exons.

**Table 2: Human marker genes and their location within a specific SRO.**

| SRO | Accession | Description |
|---|---|---|
| SRO_8p_1 | | |
| | NM_033225 | CUB and Sushi multiple domains 1 (CSMD1), mRNA. |
| SRO_8p_2 | | |
| | NM_003747 | tankyrase, TRF1-interacting ankyrin-related ADP-ribose polymerase (TNKS), mRNA. |
| | NM_017884 | PIN2-interacting protein 1 (PINX1), mRNA. |
| | NM_004462 | farnesyl-diphosphate farnesyltransferase 1 (FDFT1), mRNA. |
| | NM_006765 | Putative prostate cancer tumor suppressor (N33), mRNA. |
| | NM_016353 | zinc finger, DHHC domain containing 2 (ZDHHC2), mRNA. |
| | NM_152415 | hypothetical protein FLJ32642 (FLJ32642), mRNA. |
| | NM_020749 | AT2 receptor-interacting protein 1 (ATIP1), mRNA. |
| | NM_006197 | pericentriolar material 1 (PCM1), mRNA. |
| | NM_004315 | N-acylsphingosine amidohydrolase (acid ceramidase) 1 (ASAH1), mRNA. |
| | NM_000662 | N-acetyltransferase 1 (arylamine N-acetyltransferase) (NAT1), mRNA. |
| | NM_000015 | N-acetyltransferase 2 (arylamine N-acetyltransferase) (NAT2), mRNA. |
| | NM_001715 | B lymphoid tyrosine kinase (BLK), mRNA. |
| | NM_012331 | methionine sulfoxide reductase A (MSRA), mRNA. |
| | NM_139167 | sarcoglycan zeta (SGCZ), mRNA. |
| | NM_013354 | CCR4-NOT transcription complex, subunit 7 (CNOT7), transcript variant 1, mRNA. |
| SRO_8p_3 | | |
| | | |
| | D31887 | mRNA for KIAA0062 gene, partial cds. |
| | NM_005144 | hairless (HR), transcript variant 1, mRNA. |
| | | |
| SRO_8q_2 | | |
| | NM_138455 | collagen triple helix repeat containing 1 (CTHRC1), mRNA. |
| | | |
| SRO_8q_3 | | |
| | AL713790 | cDNA DKFZp564F1062 (from clone DKFZp564F1062). |
| | | |
| SRO_8q_4 | | |
| | BC030520 | similar to hypothetical protein F33H2.2 - aenorhabditis elegans, clone MGC:40403 IMAGE:5180533, mRNA, complete cds. |
| | | |
| SRO_8q_6 | | |
| | NM_032611 | protein tyrosine phosphatase type IVA, member 3 (PTP4A3), transcript variant 1, mRNA. |
| | NM_024035 | hypothetical protein MGC3113 (MGC3113), mRNA. |
| | NM_032862 | tigger transposable element derived 5 (TIGD5), mRNA. |
| | NM_017767 | solute carrier family 39 (zinc transporter), member 4 (SLC39A4), mRNA. |
| | NM_002346 | lymphocyte antigen 6 complex, locus E (LY6E), mRNA. |
| | AF289596 | clone pp7882 unknown mRNA. |
| | NM_012162 | F-box and leucine-rich repeat protein 6 (FBXL6), transcript variant 1, mRNA. |
| | AB051475 | mRNA for KIAA1688 protein, partial cds. |
| | | |
| SRO_13q_1 | | |
| | NM_001260 | cyclin-dependent kinase 8 (CDK8), mRNA. |
| | NM_006646 | WAS protein family, member 3 (WASF3), mRNA. |
| | | |
| SRO_13q_2 | | |
| | U50531 | BRCA2 region, mRNA sequence CG030. |
| | NM_145293 | similar to hypothetical protein FLJ20897 (LOC 196549), mRNA. |
| | U50524 | BRCA2 region, mRNA sequence CG017. |
| | NM_033111 | CG016 (LOC88523), mRNA. |
| | | |
| SRO_13q_3 | | |
| | NM_024808 | hypothetical protein FLJ22624 (FLJ22624), mRNA. |
| | NM_014832 | TBC1 domain family, member 4 (TBC1D4), mRNA. |
| | NM_006002 | ubiquitin carboxyl-terminal esterase L3 (ubiquitin thiolesterase) (UCHL3), mRNA. |
| | NM_005358 | LIM domain only 7 (LM07), transcript variant 1, mRNA. |
| | NM_012158 | F-box and leucine-rich repeat protein 3A (FBXL3A), mRNA. |
| | NM_015057 | KIAA0916 protein (KIAA0916), mRNA. |
| | NM_024546 | hypothetical protein FLJ13449 (FLJ13449), mRNA. |
| | BC026126 | hypothetical protein KIAA1165, clone MGC:3415 IMAGE:3027907, mRNA, complete cds. |
| | NM_006493 | ceroid-lipofuscinosis, neuronal 5 (CLN5), mRNA. |
| | | |
| SRO_13q_6 | | |
| | NM_017817 | RAB20, member RAS oncogene family (RAB20), mRNA. |
| | NM_018210 | hypothetical protein FLJ10769 (FLJ10769), mRNA. |
| | NM_017664 | hypothetical protein FLJ20093 (FLJ20093), mRNA. |
| | NM_003899 | Rho guanine nucleotide exchange factor (GEF) 7 (ARHGEF7), transcript variant 1, mRNA. |
| | NM_003903 | CDC16 cell division cycle 16 homolog (S. cerevisiae) (CDC16), mRNA. |
| | NM_018386 | hypothetical protein FLJ11305 (FLJ11305), mRNA. |
| | BC008975 | clone MGC:16774 IMAGE:4215274, mRNA, complete cds. |
| | NM_023011 | similar to yeast Upf3, variant A (UPF3A), transcript variant 1, mRNA. |
| | | |
| SRO_15q_3 | | |
| | NM_033240 | promyelocytic leukemia (PML), transcript variant 2, mRNA. |
| | NM_004255 | cytochrome c oxidase subunit Va (COX5A), nuclear gene encoding mitochondrial protein, mRNA. |
| | NM_000126 | electron-transfer-flavoprotein, alpha polypeptide (glutaric aciduria II) (ETFA), nuclear gene encoding mitochondrial protein, mRNA. |
| | NM_002573 | platelet-activating factor acetylhydrolase, isoform Ib, gamma subunit 29kDa (PAFAH1B3), mRNA. |
| | AB033025 | mRNA for KIAA1199 protein, partial cds. |
| | NM_030574 | START domain containing 5 (STARD5), mRNA. |
| | NM_015079 | KIAA1055 protein (KIAA1055), mRNA. |
| | NM_015969 | mitochondrial ribosomal protein S17 (MRPS17), nuclear gene encoding mitochondrial protein, mRNA. |
| | NM_016073 | likely ortholog of mouse hepatoma-derived growth factor, related protein 3 (HDGFRP3), mRNA. |
| | | |
| SRO_17p_1 | | |
| | NM_130766 | skeletal muscle and kidney enriched inositol phosphatase (SKIP), transcript variant 2, mRNA. |
| | NM_015721 | gem (nuclear organelle) associated protein 4 (GEMIN4), mRNA. |
| | NM_031430 | rab interacting lysosomal protein (RILP), mRNA. |
| | | |
| SRO_18q_2 | | |
| | NM_004715 | CTD (carboxy-terminal domain, RNA polymerase II, polypeptide A) phosphatase, subunit 1 (CTDP1), transcript variant FCP1a, mRNA. |
| | NM_006701 | similar to S. pombe dim1+ (DIM1), mRNA. |
| | NM_014913 | KIAA0863 protein (KIAA0863), mRNA. |
| | | |
| SRO_20q_1 | | |
| | NM_006892 | DNA (cytosine-5-)-methyltransferase 3 beta (DNMT3B), mRNA. |
| | NM_012325 | microtubule-associated protein, RP/EB family, member 1 (MAPRE1), mRNA. |
| | NM_005225 | E2F transcription factor 1 (E2F1), mRNA. |
| | NM_000687 | S-adenosylhomocysteine hydrolase (AHCY), mRNA. |
| | NM_014183 | dynein, cytoplasmic, light polypeptide 2A (DNCL2A), mRNA. |
| | NM_080476 | CDC91 cell division cycle 91-like 1 (S. cerevisiae) (CDC91L1), mRNA. |
| | BC035639 | clone MGC:46653 IMAGE:5547220, mRNA, complete cds. |
| | NM_014071 | nuclear receptor coactivator 6 (NCOA6), mRNA. |
| | NM_018677 | acetyl-Coenzyme A synthetase 2 (ADP forming) (ACAS2), transcript variant 1, mRNA. |
| | NM_002212 | integrin beta 4 binding protein (ITGB4BP), mRNA. |
| | NM_018244 | chromosome 20 open reading frame 44 (C20orf44), mRNA. |
| | NM_006047 | RNA binding motif protein 12 (RBM12), transcript variant 1, mRNA. |
| | NM_021100 | NFS1 nitrogen fixation 1 (S. cerevisiae) (NFS1), mRNA. |
| | NM_016436 | chromosome 20 open reading frame 104 (C20orf104), mRNA. |
| | NM_015511 | chromosome 20 open reading frame 4 (C20orf4), mRNA. |
| | NM_014902 | KIAA0964 protein (KIAA0964), mRNA. |
| | NM_006097 | myosin regulatory light chain 2, smooth muscle isoform (MYRL2), mRNA. |
| | NM_021809 | TGFB-induced factor 2 (TALE family homeobox) (TGIF2), mRNA. |
| | NM_018840 | putative Rab5-interacting protein (RIP5), mRNA. |
| | NM_032013 | NDRG family member 3 (NDRG3), mRNA. |
| | NM_016082 | CDK5 regulatory subunit associated protein 1 (CDK5RAP1), mRNA. |
| | NM_007238 | peroxisomal membrane protein 4, 24kDa (PXMP4), mRNA. |
| | NM_003908 | eukaryotic translation initiation factor 2, subunit 2 beta, 38kDa (EIF2S2), mRNA. |
| | | |
| SRO_20q_2 | | |
| | NM_003600 | serine/threonine kinase 6 (STK6), mRNA. |
| | NM_003610 | RAE1 RNA export 1 homolog (S. pombe) (RAE1), mRNA. |
| | NM_017495 | RNA-binding region (RNP1, RRM) containing 1 (RNPC1), mRNA. |
| | NM_020182 | transmembrane, prostate androgen induced RNA |
| | | (TMEPAI), mRNA. |
| | NM_004738 | VAMP (vesicle-associated membrane protein)-associated protein B and C (VAPB), mRNA. |
| | NM_153360 | hypothetical protein FLJ90166 (FLJ90166), mRNA. |
| | NM_080425 | GNAS complex locus (GNAS), transcript variant 3, mRNA. |
| | NM_016397 | TH1-like (Drosophila) (TH1L), mRNA. |
| | NM_006886 | ATP synthase, H+ transporting, mitochondrial F1 complex, epsilon subunit (ATP5E), nuclear gene encoding mitochondrial protein, mRNA. |
| | NM_016045 | chromosome 20 open reading frame 45 (C20orf45), mRNA. |
| | NM_000114 | endothelin 3 (EDN3), mRNA. |
| | NM_020673 | RAB22A, member RAS oncogene family (RAB22A), mRNA. |
| | | |
| SRO_20q_3 | | |
| | NM_003185 | TAF4 RNA polymerase II, TATA box binding protein (TBP)-associated factor, 135kDa (TAF4), mRNA. |
| | NM_014054 | chromosome 20 open reading frame 40 (C20orf40), mRNA. |
| | NM_152255 | proteasome (prosome, macropain) subunit, alpha type, 7 (PSMA7), transcript variant 2, mRNA. |
| | NM_007002 | adhesion regulating molecule 1 (ADRM1), mRNA. |
| | BC003122 | clone IMAGE:3357127, mRNA, partial cds. |
| | NM_016354 | solute carrier family 21 (organic anion transporter), member 12 (SLC21A12), mRNA. |
| | NM_018270 | chromosome 20 open reading frame 20 (C20orf20), mRNA. |
| | NM_001853 | collagen, type IX, alpha 3 (COL9A3), mRNA. |
| | NM_006602 | transcription factor-like 5 (basic helix-loop-helix) (TCFL5), mRNA. |
| | NM_022105 | death associated transcription factor 1 (DATF1), transcript variant 1, mRNA. |
| | NM_017896 | chromosome 20 open reading frame 11 (C20orf11), mRNA. |
| | NM_022082 | chromosome 20 open reading frame 59 (C20orf59), mRNA. |
| | NM_015666 | GTP binding protein 5 (putative) (GTPBP5), mRNA. |
| | NM_144498 | oxysterol binding protein-like 2 (OSBPL2), transcript variant 2, mRNA. |
| | NM_017798 | chromosome 20 open reading frame 21 (C20orf21), mRNA. |
| | NM_012384 | glucocorticoid modulatory element binding protein 2 (GMEB2), mRNA. |
| | NM_032527 | hypothetical protein FLJ14972 (KIAA1847), mRNA. |
| | BC025345 | Similar to LOC149651, clone MGC:39393 IMAGE:4862156, mRNA, complete cds. |
| | NM_033405 | peroxisomal proliferator-activated receptor A interacting complex 285 (PRIC285), mRNA. |

In particular embodiments the detection is performed on a part of a gene (comprising the detection of at least a part of an intron or an exon of gene). In further particular embodiments the gene is an oncogene. An oncogene is a gene within a region of gain which is overexpressed due to this gain, and of which the function suggests a role in cancer (e.g. transcription factor, cells cycle genes and the like). Examples of such genes, located in chromosome 20q are C20orf24, AURKA, RNPC1, TH1L, ADRM1, C20orf20 and TCFL5.

Accordingly, in a further aspect, the present invention provides primer pairs or probes for the specific detection of the marker genes of the present invention, more particularly the marker genes of Table 2. The present invention further provides sets of primers or probes comprising combinations of primers or probes for the specific detection of one or more marker genes, e.g. for a set of marker genes comprising one gene for each chromosomal aberration associated with colorectal adenocarcinoma. Further embodiments relate to combinations of primers or probes for a marker gene located within each of the SRO identified in Table 1. Further particular embodiments relate to primers/probes or sets of primers/probes for the detection of one or more marker genes which are overexpressed in adenocarcinoma cells compared to adenoma cells. Further particular embodiments relate to primers/probes or sets of primers/probes for the detection of one or more oncogenes selected from Table 2. According to one embodiment, the primers according to this aspect of the invention are particularly suited for quantitative PCR. According to a further embodiment, the specific probes of the present invention are particularly suited for screening genomic material in an array.

Particular and preferred aspects of the invention are set out in the accompanying independent and dependent claims. Features from the dependent claims may be combined with features of the independent claims and with features of other dependent claims as appropriate and not merely as explicitly set out in the claims.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1: Determination of smallest regions of overlap within the region of chromosomal gain of 20q

### Selection and preparation of tumour samples

Forty-one formalin-fixed and paraffin-embedded progressed adenomas (in which a focus of carcinoma is already present, also referred as malignant polyps) and 73 snapped frozen colorectal tumours (37 non-progressed adenomas and 36 carcinomas) were collected prospectively at the VU - University Medical Center (VUmc), Amsterdam, The Netherlands. All samples were used in compliance with the institution's ethical regulations.

The 41 malignant polyps (archival material) corresponded to 38 patients (19 females and 19 males) as three patients presented more than one malignant polyp. Mean age of the patients was 67 (range 45-86). From these polyps, the adenoma and carcinoma components were analysed separately adding to a total of 82 archival samples analysed (4 1 x2).

The 73 frozen specimens corresponded to 65 patients (31 females and 34 males). From these, 6 patients had multiple tumours: 4 patients, multiple adenomas and 2 patients, 1 or more adenomas next to a carcinoma. The mean age of the patients was 69 (range 47-89).

Array-CGH was performed in the two sets of samples and expression microarrays were done only on the frozen set.

### Isolation of DNA

DNA from archival specimens was obtained as described in detail previously (Hermsen et al. (2002) Gastroenterology, 123, 1109-1119). DNA from snap-frozen tissues was isolated with TRIzol reagent (Invitrogen, Breda, NL) following the supplier's instructions. DNA concentration and purity were measured in a Nanodrop ND-1000 spectrophotometer (Isogen, IJsselstein, NL) and integrity was evaluated in a 1% agarose gel, stained with ethidium bromide.

### Array CGH

### a) Generation of an array for comparative genomic hybridisation (CGH)

A full-genome BAC array printed in-house containing approximately 5000 DNA clones was used. The array comprised the Sanger 1Mb clone set with an average resolution along the whole genome of 1.0 Mb, the OncoBac set (Caltech), containing approximately 600 clones corresponding to 200 cancer-related genes, and selected clones of interest obtained from the Children's Hospital Oakland Research Institute (CHORI) and/or from the tilepath clone collection from Sanger to fill any gaps larger than 1 Mb on chromosome 6 and to have full-coverage contigs of regions on chromosomes 8q22-q23, 11q23, 13q21-q31 and 20q12-q13. Amplification of BAC clone DNA was done by ligation-mediated polymerase chain reaction (PCR) according to Snijders et al (Snijders et al. 2001) Nat Genet, 29, 263-264. All clones were printed in triplicate on Codelink^{™} slides (Amersham BioSciences, Roosendaal, NL) at a concentration of 1µg/µl, in 150mM sodium phosphate, pH 8.5, using the OmniGrid® 100 microarrayer (Genomic Solutions, Ann Arbor, MI, USA) equipped with SMP3 pins (TeleChem International, Sunnyvale, CA, USA).. After printing slides were processed according to the manufacturers protocol (Codelink^{™} slides; Amersham BioSciences, Roosendaal, NL).

### b) Labelling and Hybridisation

300 ng of tumour and reference (pool of DNA isolated from peripheral blood from 10 healthy individuals, either females or males) DNAs were labelled by random priming (Bioprime DNA Labeling System, Invitrogen, Breda, NL). Removal of unincorporated nucleotides was done with sephadex columns (ProbeQuant G-50 Micro Columns - Amersham BioSciences, Roosendaal, NL). Cy3 labelled test genomic DNA and Cy5 labelled reference DNA were combined and co- precipitated with 100 µg of human Cot-1 DNA (Invitrogen, Breda, NL) by adding 0.1 volume of 3 M sodium acetate (pH 5.2) and 2.5 volumes of ice-cold 100% ethanol. The precipitate was collected by centrifugation at 14,000 rpm for 30 minutes at 4 °C. After air-drying, the pellet was dissolved in 13 ml Yeast tRNA (100 mg/ml, Invitrogen) and 26 ml 20% SDS taking care to prevent foam formation.

After incubating at room temperature for 15 min, 91 µl of Master mix [14.3% (w/v) dextran sulphate (USB), 50% (v/v) formamide (Invitrogen), 2.9x SSC pH 7.0 (Sigma)] was added and gently mixed. The DNA samples were denatured for 10 minutes at 73 °C followed by a 60 minutes incubation at 37 °C to allow the Cot-1 DNA to block repetitive sequences. The arrays were incubated for 38h at 37°C with the denatured and blocked hybridisation mixture in a hybridisation station (HybArray12^{™} - Perkin Elmer Life Sciences, Zaventem, BE). After hybridisation, slides were washed in a solution containing 50% formamide, 2x SSC, pH 7 for 3 minutes at 45 °C, followed by 1 minute wash steps at room temperature with PN buffer (PN: 0.1 M sodium phosphate, 0.1 % nonidet P40, pH 8), 0.2 x SSC, 0.1 x SSC and 0.01 x SSC. Slides were dried by centrifugation at 1000 rpm for 3 min at room temperature.

### c) Detection

Images of the arrays were acquired by scanning (Agilent DNA Microarray scanner G2505B- Agilent technologies, Palo Alto, USA) and Imagene 5.6 software (Biodiscovery Ltd, Marina del Rey, California) was used for automatic feature extraction (segmentation of the spots and quantification of the signal and background intensities for each spot for the two channels Cy3 and Cy5). Default settings for the flagging of poor quality spots was used. A Microsoft Excel sheet was used to subtract local background from the signal median intensities of both test and reference DNA. The median of the triplicate spots was calculated for each BAC clone and 2 log ratios (tumour/normal reference signal) were normalized by subtraction of the mode value of BAC clones on chromosomes 1-22. If the standard deviation of the intensity of the three spots was greater than 0.2, clones were excluded from further analysis. Furthermore, clones with more than 20% missing values in all tumours were also excluded from further analysis.

All subsequent analyses were done considering the clone position from the UCSC July 2003 freeze of the Human Golden Path.

### d) Data analysis

### Copy number segmentation

To segment DNA copy number alterations (gains and/or losses), a smoothing algorithm "aCGH-Smooth" was applied (Jong et al. (2004) Bioinformatics, 20, 3636-3637). Smoothed 2 log ratio -0.15 and 0.15 were used as thresholds, based on 99% confidence intervals (99% CIs) obtained for 15 normal-to-normal hybridisations . Only gains and losses covering at least three consecutive BAC clones were included. Amplification was called when 2 log ratios exceeded 1.0.

DNA copy number data were stored in a modified version of ArrayCGHbase (Menten et al. (2005) BMC Bioinformatics, 6, 124). Median absolute deviation (MAD) was determined for each case as parameter to evaluate the array CGH quality. Briefly, the median of all log2 ratios from all autosomes was calculated, then the distance to the median value was calculated for each ratio and finally, the median of all the distances gives the MAD value (de Wiel, unpublished data). Only cases with MAD <0.2 were further analysed. Array-CGH profiles were visualized in ArraCGHbase and different output files were exported for downstream applications.

### Statistical analysis

Unsupervised cluster analysis was done using TIGR multi experiment viewer (TMev), applying complete linkage and using Euclidian distances as metric. For supervised analysis, comparing two groups, CGHMultiArray was used (van de Wiel et al. (2005) Bioinformatics, 21, 3193-3194.). For paired-analysis between lesions within the same tumour (adenoma and carcinoma components from within the same progressed adenoma) and adapted version of CGHMultiArray was used based on the Wilcoxon signed-rank test corrected for ties.

For defining the most frequent smallest regions of overlap (SRO) for gains on chromosome 20, throughout all the cases, STAC - Significance Testing for Aberrant Copy-number was used (Diskin et al. (2006). Genome Res. 16, 1149-1158.).

### Results

The determination of the SROs as disclosed in the present invention is illustrated in detail herein for the region of chromosomal gain at 20q. First 41 progressed adenomas (malignant polyps), which were previously analysed by CGH (Hermsen et al. (2002) cited above were analysed by array-CGH, in order to narrow down the gained region on 20q. As described therein, we analysed separately the adenoma and the carcinoma components of the malignant polyps for DNA copy number alterations. Losses of 1p, 4, 8p, 14q, 15q, 17p and 18 and gains of 1q, 6p, 7, 8q, 13q, 17q, 19p, 20q and 22q were observed in >20% of cases, of which 8p and 18 loss and 13q and 20q gains were the most frequent, occurring in more than 35% of the cases. Gain of chromosome 20 alone occurred in more than 60% of the cases. Genome wide, the pattern of copy number changes did not differ between adenoma and carcinoma components in progressed adenomas, i.e. the aberrations found in the carcinoma component were already present on the adenoma component, although with lower frequencies or amplitudes.

Next, the copy number changes of the 37 non-progressed adenomas and 36 carcinomas were analysed. From the 73 tumours, 67 (34 adenomas and 33 carcinomas) showed high quality genomic profiles (corresponding to a 8% drop-out), from which results are displayed. In adenomas the frequency of aberrations obviously was very low. In contrast, carcinomas showed frequent (>20% of cases) 1p, 4, 8p, 14q, 15q, 17p and 18 losses and 1q, 6p, 7, 8q, 13q, 17q, 19p, 20q and 22q gains, with 8p and 18 deletions and 13q and 20q gains present in more than 35% of the cases (like in the progressed adenomas). Chromosome 20 gains occurred in less than 15% of the adenomas but in more than 60% of the carcinomas, mostly affecting either the whole chromosome or the long arm, like in the progressed adenomas.

Hierarchical clustering of these 67 tumours (non-progressed adenomas and carcinomas) on DNA copy number profiles showed a clear separation of carcinomas and adenomas into two different clusters, cluster 1 and 2, respectively, with χ² test p < 0.001. In search for those DNA copy number changes that were significantly different (p<0.05) between non-progressed adenomas and carcinomas, we observed that 4q, 8p, 8q, 13q, 15q, 18 and 20 were the relevant regions, of which loci on 20q differed most significantly (p <0.00001).

In order to determine the most relevant regions within 20q, harbouring putative oncogenes with a role in colorectal cancer progression, STAC was applied to the combined set of paraffin-embedded malignant polyps (n=41) and frozen carcinomas (n=33). Analysis of these samples revealed 3 relevant regions of aberrant copy gains on 20q, one spanning 4 Mb (32-36Mb), one spanning 3 Mb (56-59Mb) and the third one spanning 2Mb (61-64Mb). These three regions (smallest regions of overlap - SRO's) still contained 80, 35 and 94 genes, respectively. This corresponds to the SROs listed in Table 1 for 20q. Similar analyses were performed for the other regions of chromosomal aberration linked with adenocarcinomas and the SROs identified for each of these regions are also provided in Table 1.

### Example 2: Microarray expression analysis

To investigate the effects of chromosomal instability on gene expression in colorectal adenoma to carcinoma progression, whole-genome copy number changes were analysed, by array-CGH, and expression levels, by microarray analysis, on a series of 68 colorectal tumours (37 non-progressed adenomas, and 31 carcinomas). Putative oncogenes that play a role in the progression of colorectal cancer are identified for the most frequently occurring type of chromosomal aberration namely 20q gain.

In Table 2 an overview is provided of the genes located within the SROs of Table 1, which were found to have a significantly different expression (FDR < 0,1 (false determination rate)) when comparing colorectal adenoma and adenocarcinoma tissue, as determined by microarray analysis of mRNA from biopsy or resection samples.

## Claims

1. An in vitro method for detecting the presence of colorectal adenocarcinoma cells in a patient, the method comprising the step of:
detecting the presence of gain or loss of at least one smallest region of overlap (SRO) in the genomic DNA of a test sample of said patient, wherein said SRO is selected from:
- the group consisting of SRO_8p_1, SRO_8p_2, SRO_8p_3 and SRO_8p_4 on chromosome 8p, and/or
- the group consisting of SRO_8q_1, SRO_8q_2, SRO_8q_3, SRO_8q_4, SRO_8q_5 and SRO_8q_6 on chromosome 8q, and/or
- the group consisting of SRO_13q_1, SRO_13q_2, SRO_13q_3, SRO_13q_4, SRO_13q_5 and SRO_13q_6 on chromosome 13 q, and/or
- the group consisting of SRO_15q_1, SRO_15q_2 and SRO_15q_3 on chromosome 15q, and/or
- the group consisting of SRO_17p_1 and SRO_17p_2 on chromosome 17p, and/or
- the group consisting of SRO_18q_1 and SRO_18q_2 on chromosome 18 , and/or
- the group consisting of SRO_20q_1, SRO_20q_2 and SRO_20q_3 on chromosome 20q,
wherein the presence of gain or loss of one or more of said SROs is indicative of the presence of colorectal adenocarcinoma cells in said patient.

2. The method according to claim 1, wherein said detection encompasses the detection of gain or loss of at least one SRO for each of the regions of chromosomal loss or gain, i.e. chromosomal gain at 8q, 13q, and 20q, and chromosomal loss at 8p, 15q, 17p and 18q.

3. The method according to claim 1, wherein said detection encompasses the detection of gain or loss for all of said SROs of at least one chromosomal region.

4. The method according to claim 1, wherein said detection encompasses the detection of gain or loss for all of said SROs of each of the listed chromosomal regions.

5. The method according to any of claims 1 to 4, wherein the detection of chromosomal gain or loss is performed using Comparative Genomic Hybridisation.

6. The method according to any of claims 1 to 4, wherein the detection of chromosomal gain or loss is performed by a quantitative PCR detection of at least part of a gene located in said one or more SROs.

7. The method according to any of claims 1 to 4, wherein the detection of chromosomal loss of a SRO is performed with a PCR reaction using a SRO-specific primer pair comprising a forward primer located 5' and a reverse primer located 3' from said SRO.

8. The method according to any of claims 1 to 4, wherein the detection of chromosomal gain of a SRO is performed with a PCR reaction using a forward primer in the 3' region of said SRO and a reverse primer in the 5' region of a said SRO.

9. The method according to any of claims 1 to 4, wherein the detection of chromosomal gain or loss of said SRO is performed using multiplex ligation- dependent probe amplification (MPLA).

10. The method according to any of claims 1 to 9, wherein said test sample is a colorectal tumour resection or biopsy.

11. The method according to any of claims 1 to 9, wherein said test sample is a stool sample.

12. The method according to any of claims 1 to 9, wherein said test sample is a sample of a tissue or fluid selected from the group consisting of blood, urine, saliva, and colon fluid.

13. Use of a method according to any of claims 1 to 12, for diagnosing the progression of a colorectal adenoma into an adenocarcinoma.

14. A kit for the detection of chromosomal deletions or duplications, said kit comprising, for one or more SROs of each of the chromosomal regions depicted in Table 1, one or more oligonucleotides hybridising specifically under stringent conditions with a sequence within said SRO or with a sequence within the genomic sequence up to about 1 Mb located 3' or 5' from the boundaries of said SRO.

15. The kit according to claim 14, wherein the one or more oligonucleotides are a pair of SRO-specific PCR primers.

16. The kit according to claim 14, wherein the one or more oligonucleotides are one or more SRO-specific probes.

17. The kit according to claim 14, wherein the one or more oligonucleotides are primer pairs which specifically amplify a nucleotide sequence within an SRO.

18. The kit according to claim 17, wherein the one or more oligonucleotides are primer pairs each of which specifically amplify a sequence of a marker gene of Table 2.

19. Use of a kit according to any one of claims 14 to 18, for the in vitro determination of a chromosomal aberration in genetic material of a colorectal lesion.

20. Use of a kit according to any one of claims 14 to 18, for the in vitro determination of the progression of a colorectal adenoma into an adenocarcinoma.
